(19) Europäisches Patentamt
European Patent Office
Office européen des brevets

(11) Veröffentlichungsnummer: **0 354 458**
**A2**

# (12) EUROPÄISCHE PATENTANMELDUNG

(21) Anmeldenummer: **89114238.2**

(51) Int. Cl.⁴: **C07D 335/16 , G03F 7/00**

(22) Anmeldetag: **02.08.89**

(30) Priorität: **09.08.88 DE 3826947**

(43) Veröffentlichungstag der Anmeldung: **14.02.90 Patentblatt 90/07**

(84) Benannte Vertragsstaaten: **DE FR GB**

(71) Anmelder: **MERCK PATENT GESELLSCHAFT MIT BESCHRÄNKTER HAFTUNG**
**Frankfurter Strasse 250 Postfach 4119**
**D-6100 Darmstadt(DE)**

(72) Erfinder: **Köhler, Manfred, Dr.**
**Hebbelstrasse 38**
**D-6100 Darmstadt(DE)**
Erfinder: **Ohngemach, Jörg, Dr.**
**Taubenstrasse 6**
**D-6107 Reinheim(DE)**

## (54) Fotoinitiatoren kombinierter Struktur.

(57) Die Erfindung betrifft Fotoinitiatoren kombinierter Struktur und ihre Anwendung als Fotoinitiatoren bei der Fotopolymerisation ethylenisch ungesättigter Verbindungen oder solche enthaltender Bindemittelsysteme.

EP 0 354 458 A2

# Fotoinitiatoren kombinierter Struktur

Die Erfindung betrifft Fotoinitiatoren mit aus unterschiedlichen Fotoinitiator-Grundstrukturtypen kombinierter Molekülstruktur. Es handelt sich hierbei um Molekülstrukturen, die im wesentlichen aus einem Strukturteil vom Thioxanthon-Typ und einem Strukturteil vom Typ der α-substituierten Alkylphenone zusammengesetzt sind. Derartige Fotoinitiatoren kombinierter Struktur dienen als Fotoinitiatoren für die Fotopolymerisation ethylenisch ungesättigter Verbindungen oder solche enthaltender Bindemittelsysteme. Sie zeigen hierbei eine Reihe vielfach unerwarteter Vorteile gegenüber den ihnen zugrundeliegenden Fotoinitiator-Einzelstrukturen bzw. deren Gemische.

Fotochemisch induzierte Polymerisationsreaktionen haben in der Technik große Bedeutung erlangt, insbesondere wenn es um eine schnelle Härtung von dünnen Schichten geht, wie z.B. bei der Härtung von Lacküberzügen und Kunststoffbeschichtungen auf Papier, Holz, Metall und Kunststoff oder bei der Trocknung von Druckfarben. Dabei zeichnet sich die Strahlungshärtung in Gegenwart von Fotoinitiatoren gegenüber konventionellen Methoden zum Trocknen oder Härten von Beschichtungen durch Rohstoff-und Energieersparnis, geringe thermische Belastung des Untergrundes, und insbesondere eine hohe Geschwindigkeit aus. Aber auch die Herstellung von Polymermaterialien an sich durch Polymerisation entsprechender ungesättigter monomerer Ausgangsmaterialien erfolgt vielfach fotochemisch und mit Hilfe von Fotoinitiatoren, wobei übliche Verfahren, wie Lösungs- und Emulsionspolymerisationen zur Anwendung gelangen.

Da bei den genannten Reaktionen in der Regel keiner der Reaktionspartner in der Lage ist, die fotochemisch wirksame Strahlung in ausreichendem Maße zu absorbieren, müssen Fotoinitiatoren zugesetzt werden, die in der Lage sind, entweder eingestrahlte energiereiche Strahlung, meist UV-Licht, zu absorbieren und dabei aktive Starterradikale zu bilden, die ihrerseits die Fotopolymerisation auslösen, oder die aufgenommene Energie zur Radikalbildung auf einen der polymerisierbaren Reaktionspartner zu übertragen. An der eigentlichen Polymerisationsreaktion nehmen die Initiatoren normalerweise nicht teil.

Hierfür geeignete Fotoinitiatoren sind überwiegend Verbindungen vom Typ der aromatischen Ketone, wie Benzophenon, Benzil oder Thioxanthone sowie vom Typ der α-substituierten Alkylphenone, wie Benzoinether, Benzilmonoketale, Dialkoxyacetophenone oder Hydroxyalkylphenone, sowie von diesen Strukturtypen abgeleitete Derivate.

Die fortschreitende Diversifizierung und Spezialisierung von Verfahren und Produkten auf dem Sektor der Beschichtungstechnik mit Polymermaterialien und das vermehrt.einhergehende Erfordernis von maßgeschneiderten Problemlösungen bringt es mit sich, daß auch an Fotoinitiatoren immer höhere und spezifischere Anforderungen gestellt werden. Fotoinitiatoren konventioneller Art erfüllen daher vielfach nicht oder zumindest nicht optimal die heute an sie gestellten Forderungen.

Wesentliche Problemkreise sind hierbei die Erzielung einer maximalen Fotoinitiator-Wirkung, vor allem auch in pigmentierten Systemen, die Kompatibilität mit den unterschiedlichsten Bindemittelsystemen, ihren reaktiven Bestandteilen und sonstigen modifizierenden Zusätzen, die Dunkellagerstabilität der mit dem Inititator versehenen Systeme und die möglichen Qualitätsbeeinträchtigungen des gehärteten Endproduktes, wie Vergilbung, durch unumgesetzte Initiatorreste und Inititiatorabbauprodukte.

Fotoinititatoren vom Typ der aromatischen Ketone, wie insbesondere Benzophenon und Thioxanthon sowie deren Derivate, gehören bekanntlich zur Gruppe der "intermolekularen H-Abstraktoren", die Fotoinitiatoren vom Typ der α-substituierten Alkylphenone, wie α-Hydroxy- und α-Aminoalkylphenone, gehören zur Gruppe der "intramolekularen α-Spalter". Intramolekulare α-Spalter zerfallen nach der Strahlungsanregung und Übergang in den angeregten Triplett-Zustand in zwei aktive Starterradikale. Intermolekulare H-Abstraktoren benötigen für eine effektive Wirksamkeit Coinitiatoren, meist werden hierfür Amine eingesetzt, von denen sie im angeregten Triplett-Zustand ein Wasserstoffatom abstrahieren und hierdurch die aktiven Starterradikale bilden.

Von den zahlreichen bekannten Fotoinitiatoren haben sich die Hydroxyalkylphenon-Fotoinitiatoren, wie sie in den Patentschriften DE-PS 27 22 264 und EP-PS 3002 beschrieben sind, als besonders vorteilhaft erwiesen, vor allem aufgrund ihrer hohen Reaktivität aber auch wegen der vorzüglichen Dunkellagerstabilität der mit ihnen versetzten strahlungshärtbaren Systeme sowie der geringen Neigung der damit gehärteten Schichten zu vergilben. Diese Verbindungen absorbieren jedoch vornehmlich im mittleren bis niederen UV-Wellenlängenbereich und sind daher für pigmentierte Systeme weniger günstig. Gerade bei mit Titandioxid pigmentierten Systemen wird ein großer Teil der aktiven Wellenlänge durch das Pigment absorbiert, so daß höhere Initiatorkonzentrationen und/oder zusätzliche spektrale Sensibilisatoren eingesetzt werden müssen.

Thioxanthon-Fotoinitiatoren absorbieren im längerwelligen UV-Bereich, insbesondere auch in einem "Absorptionsfenster" von Titandioxid, so daß sie gerade in dieser Hinsicht für mit Titandioxid pigmentierte Systeme prädestiniert sind. Thioxanthone haben aber den Nachteil, daß sie überwiegend schwer löslich und

nur mit Coinitiator ausreichend aktiv sind. Systeme mit Thioxanthon-Amin-Gemischen neigen darüber hinaus sehr stark zu vergilben, was gerade bei weißpigmentierten Systemen sehr störend ist.

Als günstiger für pigmentierte Systeme haben sich Gemische aus Hydroxyalkylphenonen und Thioxanthonen erwiesen. Die höhere Reaktivität derartiger Gemische gegenüber den reinen Hydroxyalkylphenonen beruht wahrscheinlich auf einer gewissen spektralen Sensibilisierung im längerwelligen Bereich durch das Thioxanthon.

Die bekannten Fotoinitiatoren und Fotoinitiatorkombinationen entsprechen aber vielfach nicht ausreichend den heute an sie gestellten gesteigerten Anforderungen. Der Erfindung lag daher die Problemstellung zugrunde, leistungsfähigere Fotoinitiatoren aufzufinden und bereitzustellen, die vor allem auch für pigmentierte Systeme geeignet sind.

Es wurde nun gefunden, daß diese Anforderungen in hervorragender Weise erfüllt werden, von Verbindungen der Formel I

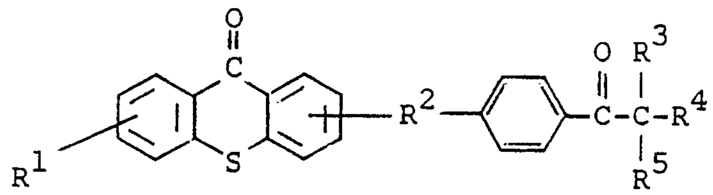

(I)

worin

$R^1$ Wasserstoff, Halogen, $C_{1-6}$-Alkyl, $C_{1-6}$-Alkoxy, $C_{1-6}$-Alkylthio, $NO_2$

$R_2$ COO-$(CH_2)_m$-X oder X$(-CH_2)_m$-Y-CO-$(CH_2)_n$-X mit X gleich O, S und Y gleich O, NH, m die Zahlen 2 bis 10 und n die Zahlen 1 bis 10

$R_3$, $R_4$ jeweils unabhängig voneinander H, $C_{1-12}$-Alkyl, $C_{2-12}$-Alkenyl oder zusammen $C_{2-6}$-Alkylen

$R^5$ $OR^6$, $N(R^6)_2$,

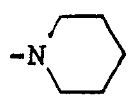 , 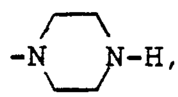 , 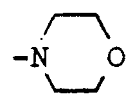 ,

$SO_2R^7$, $OSO_2R^7$

$R^6$ H, $C_{1-6}$-Alkyl oder $C_{1-6}$-Alkanoyl

$R^7$ $C_{1-6}$-Alkyl oder gegebenenfalls jeweils mit Halogen, $C_{1-6}$-Alkyl oder $C_{1-6}$-Alkoxy substituiertes Phenyl oder Benzyl

bedeuten.

Gegenstand der Erfindung sind somit die Verbindungen der Formel I.

Gegenstand der Erfindung ist weiterhin die Verwendung der Verbindungen der Formel I als Fotoinitiatoren für die Fotopolymerisation ethylenisch ungesättigter Verbindungen oder solche enthaltender Bindemittelsysteme.

Gegenstand der Erfindung sind darüber hinaus fotopolymerisierbare Bindemittelsysteme, enthaltend mindestens eine ethylenisch ungesättigte fotopolymerisierbare Verbindung sowie gegebenenfalls weitere bekannte und übliche Zusatzstoffe, die darüber hinaus mindestens eine Verbindung der Formel I als Fotoinitiator enthalten.

Gegenstand der Erfindung ist schließlich ein Verfahren zur Herstellung einer strahlungsgehärteten Beschichtung auf einem Substrat, wobei man dieses mit einem fotopolymerisierbaren Bindemittelsystem, das mindestens eine Verbindung der Formel I als Fotoinitiator enthält, beschichtet und die Härtung durch Bestrahlen mit UV-Licht einer Wellenlänge zwischen 250 und 450 nm vornimmt.

Die Verbindungen der Formel I sind neu. Es handelt sich hierbei um Fotoinitiatoren mit aus zwei unterschiedlichen Fotoinitiator-Grundstrukturtypen, nämlich vom Thioxanthontyp einerseits und vom Typ der α-substituierten Alkylphenone andererseits, kombinierter Molekülstruktur.

In Formel I wird ein Thioxanthonrest, der gegebenenfalls einen Substituenten $R^1$,der Halogen, wie insbesondere Chlor, $C_{1-6}$-Alkyl, $C_{1-6}$-Alkoxy, $C_{1-6}$-Alkylthio oder $NO_2$ darstellen kann, trägt, über eine Brückengruppierung $R^2$ mit dem aromatischen Ring eines α-substituierten Alkylphenonrestes verknüpft. Die Anbindung der Brückengruppierung $R^2$ an die Thioxanthonstruktureinheit kann in 1-, 2-, 3- oder 4-Stellung, bevorzugt in 1-, 2- oder 3-Stellung, insbesondere in 1- oder 3-Stellung, erfolgen. Im Alkylphenonrest können die Substituenten $R^3$ und $R^4$ am zur Carbonylgruppe α-ständigen C-Atom Wasserstoff, $C_{1-12}$-Alkyl, $C_{2-12}$-

Alkenyl oder zusammen $C_{2-6}$-Alkylen bedeuten. Vorzugsweise sind $R^3$ und $R^4$ jeweils Methyl. Der Substituent $R^5$ kann eine Hydroxy-, eine $C_{1-6}$-Alkoxy-, eine $C_{1-6}$-Alkanoyl-Gruppe, eine Dialkylamino- oder Dialkanoylaminogruppe, in denen die Alkyl- bzw. Alkanoylgruppen jeweils bis zu 6 C-Atomen aufweisen können, eine N-Piperidino-, eine N-Piperazino- oder eine N-Morpholinogruppe sein. Bevorzugte Fotoinitiator-Struktureinheiten hierbei sind solche, in denen $R^5$ für die Hydroxy- oder N-Morpholinogruppe steht. Besonders bevorzugt sind Fotoinitiator-Struktureinheiten vom Hydroxyalkylphenontyp. Als Brükkengruppierung $R^2$ fungieren Alkylengruppen sowie Gruppierungen, die aus mittels Ester- oder Amidgruppen verknüpften Alkylengruppen bestehen, welche ihrerseits wiederum über Carboxyl-, Sauerstoff- oder Schwefelatome mit den Fotoinitiator-Struktureinheiten verknüpft sind. Bevorzugte Brückengruppierungen sind Carbonyloxyethylenoxy-, Carbonyloxyethylenthio-, Oxymethylencarbonyloxyethylenthio- und Oxymethylencarbonylamidoethylenthiogruppen.

Typische Beispiele für Verbindungen der Formel I sind

1-(1-Thioxanthonylcarbonyloxy)-2-[4-(2-hydroxy-2-methyl)propanoylphenoxy]ethan

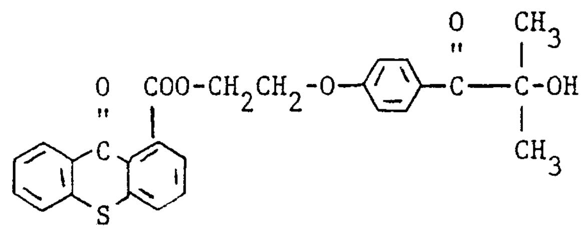

(Ia)

1-(1-Thioxanthonylcarbonyloxy)-2-[4-(2-hydroxy-2-methyl)propanoylphenylthio]ethan

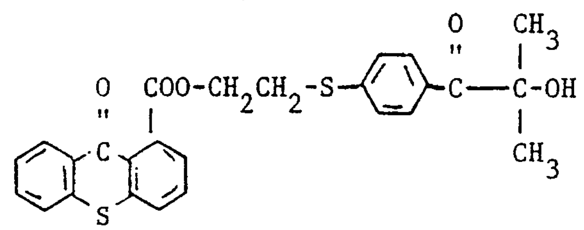

(Ib)

1-(1-Thioxanthonylcarbonyloxy)-2-[4-(2-N-morpholino-2-methyl)propanoylphenoxy]ethan

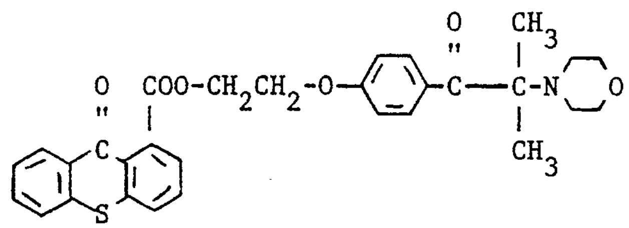

(Ic)

1- 1-Thioxanthonylcarbonyloxy)-2-[4-(2-N-morpholino-2-methyl)propanoylphenylthio]ethan

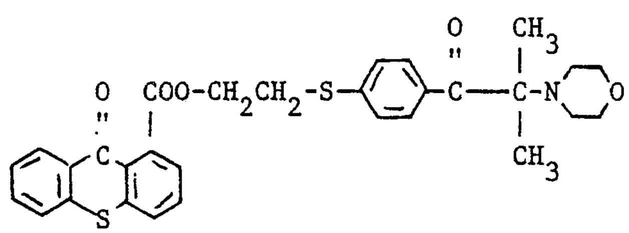

(Id)

1-(3-Thioxanthonylthio)-2-[4-(2-hydroxy-2-methyl)propanoylphenoxymethylencarbonyloxy]ethan

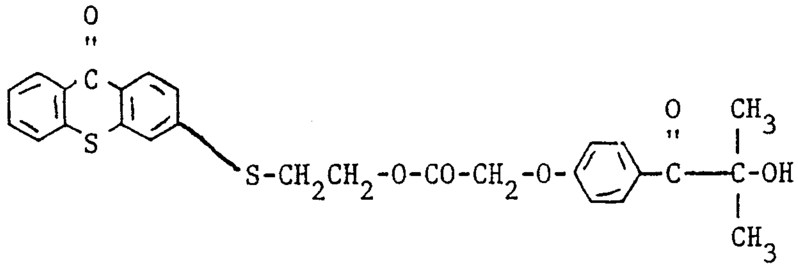

(Ie)

1-(3-Thioxanthonylthio)-2-[4-(2-hydroxy-2-methyl)propanoylphenoxymethylencarbonylamido]ethan

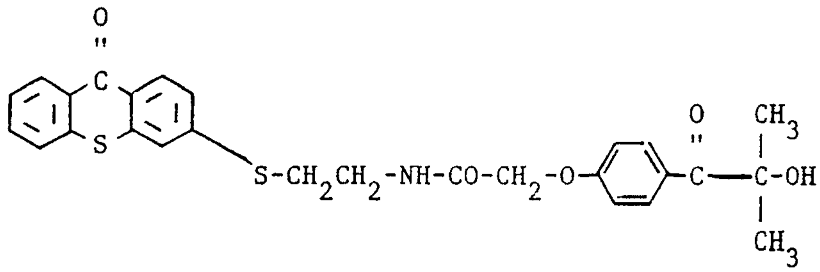

(If)

Die Herstellung der Verbindungen der Formel I erfolgt mit Hilfe gängiger Reaktionen, wie insbesondere Veresterungsreaktionen, aus entsprechend funktionalisierten Derivaten der zugrundeliegenden Thioxanthon- und Alkylphenon-Einheiten.

Die Reaktionsbedingungen hierbei entsprechen dem Üblichen und können den Standardwerken der präparativen organischen Chemie entnommen werden, z.B. HOUBEN-WEYL, Methoden der organischen Chemie, Georg-Thieme Verlag, Stuttgart, oder ORGANIC SYNTHESIS, J. Wiley, New York London Sydney.

Als funktionalisierte Derivate der Fotoinitiatorgrundstrukturen sind solche bevorzugt, die bereits die spätere Brückengruppierung $R^2$ bzw. entsprechende Vorstufen oder Teilstücke hiervon im Molekül enthalten. Entsprechende Verbindungen sind etwa mit Hydroxyethoxy-, Hydroxyethylthio-, Hydroxycarbonyl- oder Hydroxycarbonylmethylenoxy-Gruppen versehene Thioxanthon- bzw. Alkylphenon-Derivate. Funktionalisierte Fotoinitiator-Derivate dieser Art sind in den deutschen Patentanmeldungen P 37 07 891 und P 37 38 567 ausführlich beschrieben. So lassen sich beispielsweise durch Umsetzung von Thioxanthon-1-carbonsäure mit Hydroxyethoxy- oder Hydroxyethylthio-Derivaten entsprechender Alkylphenone oder deren Methosulfate die Verbindungen Ia bis Id erhalten. Durch Umsetzung von 3-Hydroxyethoxy- bzw. 3-Hydroxyethylthio thioxanthon oder deren Methosulfate mit Hydroxycarbonylethylenoxy-funktionalisierten Alkylphenonen erhält man die Verbindungen Ie und If.

Die erfindungsgemäßen Fotoinitiatoren kombinierter Struktur können sehr vorteilhaft als Fotoinitiatoren für die Fotopolymerisation ethylenisch ungesättigter Verbindungen bzw. solche enthaltender Bindemittelsysteme eingesetzt werden. Vornehmlich dienen sie analog zu konventionellen Fotoinitiatoren als UV-Härter für Lack- oder Polymerbeschichtungen sowie Druckfarben auf Basis UV-härtbarer Bindemittel- oder Hybridbindemittelsysteme, die auch in Form wäßriger Dispersionen vorliegen können. Die Anwendung erfolgt in üblicher Weise, wobei sie den Bindemittelsystemen in der Regel in Mengen von 0,1 bis 20 Gew.%, vorzugsweise 1 bis 10 Gew.% zugesetzt werden. Dieser Zusatz geschieht in der Regel durch direktes Einrühren, gegebenenfalls unter Zuhilfenahme einer untergeordneten Menge eines kompatiblen Lösungsmittels. Hierbei zeigen die Verbindungen der Formel I sehr günstige Eigenschaften in Bezug auf ihre Löslichkeit bzw. homogene Mischbarkeit mit praktisch allen üblichen strahlungshärtbaren Systemen sowie in Bezug auf die Dunkellagerstabilität der mit ihnen versetzten Systeme.

Ein besonderer Vorteil ist die überraschend höhere Reaktivität der erfindungsgemäßen Fotoinitiatoren kombinierter Struktur gegenüber den entsprechenden zugrundeliegenden Fotoinitiatortypen und deren Gemische. Dies trifft insbesondere auch für pigmentierte Systeme zu. So lassen sich mit den Verbindungen der Formel I bei äquivalentem Mengeneinsatz und vergleichbaren Härtungsbedingungen höhere Schichthärten erzielen als mit Gemischen aus Thioxanthon- und Hydroxyalkylphenon-Fotoinitiatoren. Eine Erklärung hierfür könnte in einer unmittelbaren sensibi lisierenden und/oder coinitiierenden Wechselwirkung der unterschiedlichen Initiatorgruppen, bedingt durch die räumlich nahe Fixierung im Molekül, gesehen werden.

Unter einem fotopolymerisierbaren Bindemittelsystem wird üblicherweise ein Gemisch verstanden, das mindestens eine ethylenisch ungesättigte, durch freie Radikale fotopolymerisierbare Verbindung sowie

gegebenenfalls weitere übliche Zusatzstoffe enthält. Als radikalisch polymerisierbare Komponenten sind praktisch alle Materialien, die olefinisch ungesättigte Doppelbindungen aufweisen, geeignet. Es können dies insbesondere Monomere, Oligomere und Polymere mit jeweils zumindest einer oder zweckmäßigerweise mit mehreren ungesättigten Funktionen vom Acrylat- oder Vinyl-Typ sein. Entsprechende Materialien sind dem Fachmann in großer Fülle bekannt. Beispiele für solche Verbindungen sind Acrylverbindungen, insbesondere Acrylate von aliphatischen und aromatischen Mono- oder Polyhydroxyverbindungen, wie etwa (Meth)acrylsäure einschließlich deren Salze und Amide, (Meth)acrylnitril, (Meth)acrylsäurealkylester, Hydroxyethyl(meth)acrylat, Vinyl(meth)acrylat, Ethylenglycoldi(meth)acrylat, 1,6-Hexandioldi(meth)acrylat, Trimethylolpropandi(meth)acrylat, Pentaerythrittri(meth)acrylat, weiterhin Vinylverbindungen wie etwa Vinylchlorid, Vinylidenchlorid, Styrol, Divinylbenzol, N-Vinylpyrrolidon, N-Vinylcarbazol. Beispiele für höhermolekulare und polymere Bindemittelkomponenten sind etwa acrylierte Polyester-, Acrylat-, Epoxy-, Urethan-, Polyamid- und Siliconharze. Überwiegend sind strahlungshärtbare Bindemittelsysteme Gemische mehrerer der aufgeführten niedermolekularen und höhermolekularen ungesättigten Komponenten.

Hybridbindemittelsysteme enthalten darüber hinaus noch Komponenten, die thermisch, beispielsweise durch Polykondensation oder Polyaddition, nicht jedoch radikalisch vernetzt werden können. Beispiele hierfür sind etwa säurehärtbare Melaminharze sowie Polyurethan- oder Polyester-bildende Reaktivharze.

Die strahlungshärtbaren Bindemittelsysteme können auch in Form wäßriger Dispersionen vorliegen, deren Wasseranteil nach erfolgter Beschichtung normalerweise durch kurzen Erhitzen entfernt wird.

Die mit den Verbindungen der Formel I als Fotoinitiatoren härtbaren Bindemittelsysteme können in ihren qualitativen wie quantitativen Zusammensetzungen in weiten Bereichen variiert werden und sie können insbesondere auch weitere Bestandteile und Zusätze enthalten. Zweckmäßigerweise sollte hierbei der Anteil an reaktiven Komponenten 10 Gew.% nicht unterschreiten. Als weitere Bestandteile und Zusätze in den für den jeweiligen Zweck angebrachten und üblichen Mengen können anorganische und organische Pigmente, Farbstoffe, Füllstoffe, Verlaufsmittel, oberflächenaktive Mittel, Lichtstabilisatoren und Antioxydantien, Thixotropiemittel, Mattierungsmittel, Weichmacher, Lösungsmittel, Dispergierhilfsmittel, weitere Bindemittel und Harze, sonstige Fotoinitiatoren, spektrale Sensibilisatoren und Coinitiatoren bekannter Art, weitere thermo-bzw. photoreaktive Radikalstarter sowie Kationen- oder säurebildende Katalysatoren enthalten sein.

Die genannten Bindemittelsysteme können mit den Verbindungen der Formel I erfindungsgemäß durch Einwirkung von Strahlungsenergie, insbesondere aus dem UV-Wellenlängenbereich gehärtet werden. In bestimmten Fällen, vornehm lich bei Hybridbindemittelsystemen, ist es zweckmäßig, die Aushärtung durch gleichzeitige oder nachfolgende Zufuhr von Wärmeenergie zu fördern.

Die Fotopolymerisation erfolgt nach an sich bekannten Methoden durch Bestrahlen mit Licht oder UV-Strahlung des Wellenlängenbereichs von 250-450 nm, vorzugsweise von 300-400 nm. Als Strahlenquellen können die üblichen Quecksilberdampf-Hochdruck-, -Mitteldruck- oder -Niederdrucklampen sowie Xenon- und Wolframlampen verwendet werden.

Die Durchführung der Fotopolymerisation unter Verwendung der erfindungsgemäßen Fotoinitiator kombinierter Struktur kann sowohl diskontinuierlich als auch kontinuierlich geschehen. Die Bestrahlungsdauer hängt von der Art der Durchführung, von der Art und Menge der eingesetzten polymerisierbaren Materialien, von Art und Konzentration der verwendeten Fotoinitiatoren und von der Intensität der Lichtquelle ab. Bei der Strahlungshärtung von Beschichtungen liegt sie üblicherweise im Bereich weniger Sekunden bis Minuten.

Die erfindungsgemäßen Verbindungen der Formel I werden bevorzugt verwendet als Fotoinitiatoren bei der UV-Härtung von dünnen Schichten wie beispielsweise Lackbeschichtungen auf allen hierfür üblichen Materialien und Trägern. Diese können vornehmlich sein Papier, Holz, textile Träger, Kunststoff und Metall. Ein wichtiges Anwendungsgebiet ist auch die Trocknung bzw. Härtung von Druckfarben und Siebdruckmaterialien, von denen letztere bevorzugt bei der Oberflächenbeschichtung bzw. -gestaltung von beispielsweise Dosen, Tuben und metallenen Verschlußkappen eingesetzt werden.

Ihre einfache Zugänglichkeit und ihre vorzüglichen anwendungstechnischen Eigenschaften, vor allem ihre hohe Reaktivität in pigmentierten Systemen, machen die erfindungsgemäßen Fotoinitiatoren kombinierter Struktur für den praktischen Einsatz besonders wertvoll.

Beispiel 1

1-(1-Thioxanthonylcarbonyloxy)-2-[4-(2-hydroxy-2-methyl)propanoylphenoxy]ethan (Ia)

Zu 0,3 g Natrium in 50 ml Methanol wurden bei Raumtemperatur unter Rühren 2,6 g (0,01 mol)

Thioxanthon-1-carbonsäure gegeben, 1 Stunde bei Rückflußtemperatur gerührt und dann das Lösungsmittel abdestilliert. Zu dem Rückstand wurden 3,0 g (0,01 mol) 4-(2-Methylsulfonyloxyethoxy)phenyl-(2-hydroxy-2-propyl)keton gegeben und 3 Stunden bei 80 °C gerührt. Nach dem Abkühlen wurde mit Wasser versetzt und das Gemisch mit Essigester extrahiert. Nach Entfernen des Lösungsmittels und Umkristallisation des Rückstandes aus Methylethylketon wurden 1,4 g an Verbindung Ia in Form eines gelblichen Kristallpulvers vom Schmelzpunkt 160 °C erhalten.

Analog wurden erhalten die Verbindungen

1-(1-Thioxanthonylcarbonyloxy)-2-[4-(2-hydroxy-2-methyl)propanoylphenylthio]ethan (Ib)

1-(1-Thioxanthonylcarbonyloxy)-2-[4-(2-N-morpholino-2-methyl)propanoylphenoxy]ethan (Ic)

1-(1-Thioxanthonylcarbonyloxy)-2-[4-(2-N-morpholino-2-methyl)propanoylphenylthio]ethan (Id) Smp. 134 °C.

Beispiel 2

1-(3-Thioxanthonylthio)-2-[4-(2-hydroxy-2-methyl)propanoylphenoxymethylencarbonyloxy]ethan (Ie)

Zu 1,2 g Kalium-t-butylat wurden 50 ml DMSO und 2,4 g (0,01 mol) 4-(Hydroxycarbonylmethoxy)-phenyl-(2-hydroxy-2-propyl)keton gegeben und 1 Stunde bei Raumtemperatur gerührt. Dann wurde auf 70 °C erhitzt und 3,7 g (0,01 mol) 3-(2-Methylsulfonyloxyethylthio)thioxanthon zugegeben und 4 Stunden bei dieser Temperatur gerührt. Durch Aufarbeitung wie in Beispiel 1 und Umkristallisation aus Toluol wurden 2,1 g an Verbindung Ie mit Schmelzpunkt 114 °C erhalten.

Analog wurde erhalten die Verbindung

1-(3-Thioxanthonylthio)-2-[4-(2-hydroxy-2-methyl)propanoylphenoxymethylencarbonylamido]ethan (If).

## Ansprüche

1. Verbindungen der Formel I

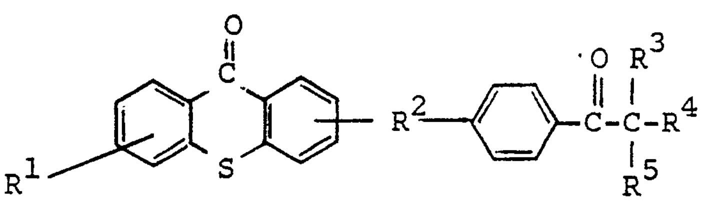

(I)

worin

$R^1$ Wasserstoff, Halogen, $C_{1-6}$-Alkyl, $C_{1-6}$-Alkoxy, $C_{1-6}$-Alkylthio, $NO_2$

$R^2$ $COO-(CH_2)_m-X$ oder $X-(CH_2)_m-Y-CO-(CH_2)_n-X$ mit X gleich O, S und Y gleich O, NH, m die Zahlen 2 bis 10 und n die Zahlen 1 bis 10

$R^3$, $R^4$ jeweils unabhängig voneinander H, $C_{1-12}$-Alkyl, $C_{2-12}$-Alkenyl oder zusammen $C_{2-6}$-Alkylen

$R^5$ $OR^6$, $N(R^6)_2$,

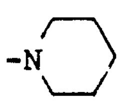

,

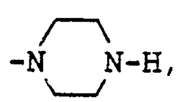

,

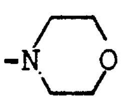

,

$SO_2R^7$, $OSO_2R^7$

$R^6$ H, $C_{1-6}$-Alkyl oder $C_{1-6}$-Alkanoyl

$R_7$ $C_{1-6}$-Alkyl oder gegebenenfalls jeweils mit Halogen, $C_{1-6}$-Alkyl oder $C_{1-6}$-Alkoxy substituiertes Phenyl oder Benzyl

bedeuten.

2. Verwendung der Verbindungen der Formel I als Fotoinitiatoren für die Fotopolymerisation ethylenisch ungesättigter Verbindungen oder solche enthaltender Bindemittelsysteme.

3. Fotopolymerisierbare Bindemittelsysteme, enthaltend mindestens eine ethylenisch ungesättigte fotopolymerisierbare Verbindung sowie gegebenenfalls weitere bekannte und übliche Zusatzstoffe, dadurch gekennzeichnet, daß sie mindestens eine Verbindung der Formel I als Fotoinitiator enthalten.

4. Fotopolymerisierbare Bindemittelsysteme nach Anspruch 3, dadurch gekennzeichnet, daß sie 0,1 bis 20 Gew.% einer Verbindung der Formel I enthält.

5. Verfahren zur Herstellung einer strahlungsgehärteten Beschichtung auf einem Substrat, wobei man dieses mit einem fotopolymerisierbaren Bindemittelsystem beschichtet und die Härtung durch Bestrahlen mit UV-Licht einer Wellenlänge zwischen 250 und 450 nm vornimmt, dadurch gekennzeichnet, daß ein Bindemittelsystem nach Anspruch 3 oder 4 verwendet wird.